# EUROPEAN PATENT APPLICATION

(11) **EP 3 156 042 A1**
(43) Date of publication of application: **19.04.2017**
(21) Application number: 16200811.4
(22) Date of filing: 30.11.2010
(51) Int. Cl.: A61K 8/81, A61K 8/92, A61Q 1/10, A61K 8/06

(54) **A COSMETIC COMPOSITION COMPRISING OIL-IN-WATER EMULSION AND MASCARA FOR EYELASHES**

(30) Priority: 30.11.2009 FR 0958464
(62) Divisional of application: 10795192.3
(71) Applicant: Natura Cosméticos S.A., 06882-700 Itapecerica da Serra- SP (BR)
(72) Inventor: GANDINI ANDREO, Luciana, 05025-010 São Paulo-SP (BR); AMARAL RODRIGUES, Adriana, 13271-220 Valinhos- São Paulo (BR); ARATANI YANO, Bárbara, 05541-030 Jardim das Vertentes-SP (BR); FACCHINI PLACERES, Patricia Helena, 13211-771 Jundiai-SP (BR)
(74) Representative: Hewson, Timothy John

(57) **Abstract**

The present invention describes a cosmetic composition comprising an oil-in-water emulsion for application on keratinic materials, of long lasting duration, that presents an ideal drying time and a crystalline network film structure that is more resistant, homogenous and flexible, allowing the application of the end product on the hairs in a cohesive, non-crumbling and uniform manner, without the formation of lumps. The cosmetic composition of long lasting duration, object of the present invention, comprises: a film forming complex comprising at least one hydrosoluble or hydrodispersible film forming agent and at least one liposoluble or lipodispersible film forming agent; a shine and performance improvement promoting complex of the end product; and a cosmetically acceptable carrier. The present invention also refers to mascaras for eyelashes or hairs comprising the aforesaid cosmetic composition.

## Description

The present invention refers to a cosmetic composition, specifically indicated for application on keratinic fibers such as head hair, eyelashes and body hair, of the emulsion type with a long lasting duration. The aforesaid composition presents a formation of a more resistant and flexible film, allowing the application of the product over the hairs in a cohesive and uniform manner.

### Background of the invention

Keratins are formed by polypeptide chains and are distinguished from other proteins by their high content of disulfide bonds (S-S) from the amino acid cysteine. These disulfide bonds form a three dimensional network with a high density of crossed links, which provide the hair with more resistance to a chemical attack. Briefly, the reduction of these links results in changes in their mechanical properties.

The eyelash has a similar structure to head hair: short and thick. These structures are located in the boundary between the eyelid and the opening of the eyeball. The upper eyelashes are longer than the lower eyelashes.

The principal function of the eyelashes is to prevent the contact of any foreign body with the eyeball, thus operating as a physical barrier and causing the act of blinking as a reflex action to any provocation. Eyelashes are terminal hairs similar to those present on the adult scalp and are basically formed of keratin.

The eyelash, similar to the hair, has four principal components: the cuticle, the cortex, the medulla and the cell membrane complex, which bind the adjacent cortical and cuticular cells.

In the cosmetic market, there are several types of mascaras for eyelashes for make-up purposes, which accordingly treat, lengthen or provide volume to the eyelashes. There also exist waterproof, black, colored and primer products, among others.

It is already known, from the prior art, mascaras for eyelashes that contain combinations of waxes, combinations of polymers and mixtures of these. However, these compositions do not emphasize refinements in relation to the application of the end product on the eyelashes. This is usually not performed in a uniform manner and, therefore, leads to the formation of lumps on these keratinic fibers.

Patent application US 2006/0159642 discloses a mascara composition for eyelashes that comprises an imide polymer with film forming properties, a coloring or pearlescent pigment, waxes and water. The aforesaid composition is an emulsion. Additionally, the aforementioned mascara for eyelashes also contains an additional film forming agent that is resistant to water. In this case, the film formed by means of the combination of the imide polymer with the PVP/eicosane copolymer do not present flexibility or a crystalline structure resistant to breaking, which could lead to the formation of lumps and crumbling.

Patent US 5,534,247 discloses a mascara composition for eyelashes containing at least one eyelash curl retention additive, which increases and sustains this effect over time. This same effect is due to the presence of a fixative resin that holds the eyelashes in a "curved" position, preventing them from being affected by humidity. The aforesaid resistance to humidity is due to the presence of a fixative resin in the formula.

Patent US 5,039,518 refers to a composition containing a powder phase and an oil phase. The aforesaid powder phase comprises spherical silica, bismuth oxychloride and mica. The oil phase comprises cosmetic waxes, ester and PVP/hexadecene copolymer, among others. The composition referred to in this document is not an O/W emulsion. It also does not present the characteristics of a film that is resistant to breakage and flexible.

Document US 2004/013624 discloses new cosmetic products that present a "special" flexibility and distending characteristics. The aforesaid product is a mascara for eyelashes that comprises an oil phase containing a non-linked polyethylene wax and at least one other wax or oil or mixtures of these; and a water phase containing a film forming agent selected from PVP/PVP-VA, polyquartenium-46 and mixtures of these. Finally, the aforesaid product contains an external film forming phase that comprises a hydrosoluble film forming agent selected from a specific group of copolymers, where the acrylates are mentioned. Despite making reference to a composition with "special" flexibility, the document does not describe a combination between copolymers of the water phase of the emulsion, copolymers of the oil phase of the emulsion and waxes, resulting in a homogenous, thick, break resistant and flexible film.

Document KR 20040066330 refers to a composition of mascara for eyelashes that forms a uniform film over the skin. The aforesaid composition comprises: a wax, such as, for example, carnauba wax; a hydrosoluble thickening agent; a film forming agent, such as a PVP/eicosane copolymer or an acrylic/acrylate copolymer, among others. In this case, it can be observed from the characteristics of the composition that the formed film is neither thick nor flexible and can, occasionally, become opaque over time, because there is no combination of specific waxes.

Patent US 5,800,825 discloses mascara formulas for anhydrous eyelashes that present a special "drying" system, which includes hardening agents. The aforesaid system comprises a mixture of an ammonium acrylate copolymer and propylene glycol, among others. The aforementioned mascara also contains water as a solvent and as a formulation carrier, waxes that act as binding and consistency agents, surfactants and PVP that acts as consistency agent, among others. The formulas described in this document are not O/W emulsions and, therefore, it is not easy to apply or remove the product due to the formation of lumps.

Document KR 20020057788 discloses compositions containing fibers in combination with at least three film forming agents, which are: at least one film soluble or dispersible in water, such as polyvinyl alcohol, acrylates and copolymers of PVP/DMAPA; at least one film forming agent viscously soluble in oil, such as polyvinyl laurate; and at least one additional film forming agent soluble or dispersible in water, which is polyvinylpyrrolidone. In this case, it can also be observed from the characteristics of the composition that the film formed is neither thick nor flexible and can, occasionally, become opaque over time, because there is no mention of a combination of specific waxes.

KR 950010934 discloses a composition of eyelash mascara that is an O/W emulsion composed of: triethanolamine, polyvinyl alcohol, amphoteric polymer of alkyl acrylate, carnauba wax and candelilla wax. However, the document does not describe a combination between copolymers of the water phase of the emulsion, copolymers of the oil phase of the emulsion and waxes, resulting in a homogenous, thick, break resistant and flexible film.

Accordingly, it can be concluded that no prior art document provides a solution to the technical problem of obtaining cosmetic compositions of the O/W emulsion type for keratinic fibers (corporeal extensions), of long duration, in order to provide the formation of a more resistant, homogenous and flexible film, accordingly allowing the application of the product over the hairs in a cohesive, uniform, non-crumbling manner, without the formation of lumps, with an ideal and versatile drying time.

### Summary of the Invention

The present invention refers to a cosmetic composition containing an oil-in-water emulsion, characterized by comprising:
a) from 1% to 25 % by weight, based on the total weight of the composition, of a film forming complex, comprising of at least one hydrosoluble or hydrodispersible film forming agent and at least one liposoluble or lipodispersible film forming agent;
b) from 8% to 15% by weight, based on the total weight of the composition, of a shine and performance improvement promoting complex; and
c) a cosmetically acceptable carrier.

The present invention also refers to a mascara for eyelashes comprising a cosmetic composition as defined above.

### Brief Description of the Drawing

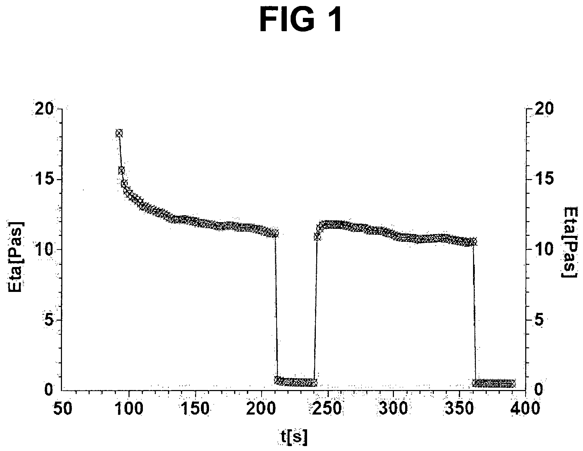

Figure 1 shows a graph with results of rheological analysis of a sample of mascara composition according to the present invention.

### Detailed description of the invention

The invention refers to a cosmetic composition of the oil-in-water emulsion type, specifically for use as mascara for eyelashes with a long lasting duration.

The emulsion should be oil-in-water because any alteration in relation to this cosmetic form interferes in the formation of the end film, its flexibility, drying time, long duration and formation of lumps and removal of the mascara.

In accordance with a preferred embodiment, the present invention refers to a cosmetic composition comprising:
a) from 1% to 25 %, preferentially from 15 to 20% by weight of a film forming complex, comprising of at least one hydrosoluble or hydrodispersible film forming agent and at least one liposoluble or lipodispersible film forming agent;
b) from 0.2% to 3.5% by weight of at least one thickener;
c) from 8% to 15% by weight of a shine and performance improvement promoting complex selected from solid lipid components; and
d) from 5% to 10% by weight of an emulsifying system; and
e) a cosmetically acceptable carrier,
all the percentages being relative to the total weight of the aforesaid cosmetic composition.

In a specific embodiment, the cosmetic composition of the oil-in-water emulsion type of the present invention is formulated for the application on the keratinic material, more specifically on the eyelashes, with a long lasting duration. The composition comprises:
I. An oil phase comprising:
   a) from 1% to 25 % by weight of a film forming complex, preferentially from 15 to 20%, comprising of at least one hydrosoluble or hydrodispersible film forming agent and at least one liposoluble or lipodispersible film forming agent;
   b) from 8% to 15% by weight of a shine and performance improvement promoting complex; and
   c) from 5% to 10% by weight of an emulsifying system;
II. A water phase that comprises:
   a) from 1% to 25 % by weight of a film forming complex, comprising of at least one hydrosoluble or hydrodispersible film forming agent and at least one liposoluble or lipodispersible film forming agent;
   b) from 0.2% to 3.5% by weight of at least one rheological agent and one thickener; and
   c) a cosmetically acceptable carrier,
all the percentages are based on the total weight of the formulation of the composition.

Generally, the polymers/copolymers conventionally employed in the formulation of cosmetics have a film formation property as a consequence of the size of the end molecule, which by not being absorbed in the skin or the hair, remains on the applied surface, covering it and forming a film. The quality of the film formed is directly related to the monomers used in the formation of the polymer, which can provide a more or less resistant film, with greater or lesser flexibility, porous or non-porous, non-resistant or resistant to water and/or wear and tear.

### Film forming complex

A first essential component of the cosmetic composition of the present invention is a film forming complex, comprised of at least one hydrosoluble or hydrodispersible film forming agent and at least one liposoluble or lipodispersible film forming agent.

The hydrosoluble or hydrodispersible film forming agent is preferentially selected from the group that consists of: acrylate copolymer, methacrylate copolymer, PVP/eicosane copolymer, sodium polyacrylate, ammonium polyacrylate, or mixtures of these. Preferentially, the hydrosoluble or hydrodispersible film forming agent is selected from acrylate copolymer, methacrylate copolymer, PVP/eicosane copolymer, or mixtures of these, or, even more preferentially, the hydrosoluble or hydrodispersible film forming agent is acrylate copolymer, which is detailed below.

### - Acrylate copolymer:

The acrylate copolymer used in the present invention is an emulsion of acrylic copolymer, free of surfactants and ethoxylates, capable of forming a fine, resistant and elastic film on the eyelash. The acrylate copolymer has a much lower vitreous transition temperature when compared to other acrylic copolymers in the market. This avoids the breakage of the film throughout the day and the formation of a sufficiently elastic film.

The acrylate copolymer is hydrosoluble and responsible for the formation of the film in the water phase. When present in the continuous phase of a formulation, which in the case of the present invention is the water phase, it provides, after drying completely, resistance to wear and tear and to water. Together with the film forming agent present in the oil phase of the emulsion, the acrylate copolymer provides a long lasting and flexibly formed film, consequently obtaining a high performance product, as well as being practical and comfortable for the user.

The liposoluble or lipodispersible film forming agent is preferably selected from the group that consists of: PVP/eicosane, trimethylsiloxysilicate and polyurethanes, or a mixture of these. Preferentially the liposoluble or lipodispersible film forming agent is the PVP/eicosane copolymer detailed below.

### - PVP/eicosane copolymer:

The PVP/eicosane copolymer is derived from the reaction of the vinylpyrrolidone with long chains of alpha-olefins. The structural representation of the copolymer in question is demonstrated below, where R can be a molecule of hydrogen or an alkyl group:

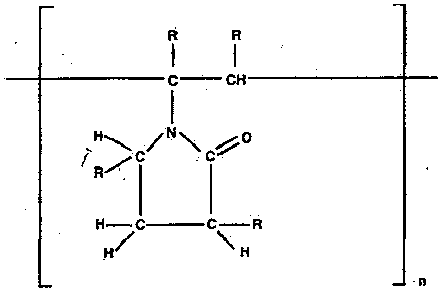

This polymer is responsible for creating resistance to wear and tear and providing the long duration of the applied film, together with the film forming agent present in the water phase of the emulsion, which is preferentially the acrylate copolymer. Furthermore, this polymer is capable of assisting in the dispersion of the pigment.

In a preferred embodiment for obtaining the cosmetic composition of the invention, a film forming complex is used that is formed by acrylate copolymer and PVP/eicosane copolymer, in a quantity that varies from 1 % to 25% of acrylate copolymer (each component should be present in a significant quantity, in other words, at least 0.5% by weight of each in relation to the total weight of the composition), preferentially from 15 to 20% by weight, based on the total weight of the composition.

### - Shine and performance improvement promoting complex of the end product

The second essential component of the composition in accordance with the present invention is a performance improvement and shine promoter complex of the end product. End "performance improvement" is understood as the system that aids to obtain and to reach the desired characteristics of the product of the present invention, thus improving their desired attributes.

The shine and performance improvement promoting complex comprises a combination of solid lipidic components selected from vegetable waxes, animal waxes or synthetic waxes. Preferentially, a combination of the selected waxes is used, from beeswax, ozokerite wax, sugar cane wax, ceresine wax, microcrystalline wax, carnauba wax and candelilla wax and a glycolic material, propylene glycol (which provides flexibility to the polymer) and polyquartenium, or mixtures thereof.

The presence of the shine and performance improvement promoting complex of the end product in the cosmetic composition in accordance with this invention, particularly when formulated as a mascara for eyelashes, provides homogeneity and greater thickness in the film formed by the film forming complex, allowing the cohesive and uniform application, without the formation of lumps and the non-crumbling of the of the product on the hairs.

Among the waxes used in the shine promoter, the following are highlighted:
- Carnauba wax:
   Carnauba wax is obtained from the palm *Copernicia cerífera* and is a hard, brittle, insipid and odorless wax. Furthermore, it is soluble in ether, benzene and turpentine and has an intense shine. Another advantage of this wax and what makes it particularly useful for the purposes of the present invention is that its harvesting does not cause environmental damage because the leaves extracted to obtain the product are naturally replaced in the following harvest.
   Carnauba wax consists of 80-85% of esters and fatty acids (alcohols and acids of long aliphatic and aromatic chains), 10-15% of fatty alcohols, 3-6% of acids and 1-3% of hydrocarbons. It also has a percentage of esterificated fatty diols, hydroxylated fatty acids and cinnamic acid (approximately 10%) in its composition. Cinnamic acid is a natural antioxidant.
   The crystallization characteristics of the carnauba wax are important in the formation of a homogenous and thick film, without the presence of lumps, which avoids the crumbling of the end product.
- Candelilla wax:
   Candelilla wax is obtained from the wild plant *Euphorbia cerifera,* which belongs to the family *Euphorbiaceae.*
   Candelilla wax is 100% vegetable, hard and brittle. Its color can vary from light brown to yellow, depending on the degree of purification. Its surface can have a high shine, which is one of the most appreciated properties of candelilla wax.
   Most of the constituents of candelilla wax are natural components found in vegetables and fruits. Its chemical composition is characterized by a high concentration of hydrocarbons (approximately 50%) and a relatively low quantity of volatile esters. Its resin content can reach up to 40% by weight, which provides the wax with adhesive properties.
   Candelilla wax is important for the mascara for eyelashes, object of the present invention, due to its crystallization characteristics. The structure obtained from its presence in the oil phase is important to guide the quantity of the product to be deposited by the brush used for the application of the cosmetic composition on the eyelash or hair. It also defines and avoids the formation of lumps that can crumble during the use of a mascara for eyelashes, for example.

In order to acquire an ideal texture and rheology for its suitable application, candelilla wax is used together with at least one thickening agent in the end formulation of the cosmetic composition object of the present invention.

In a specifically preferred embodiment, a combination of carnauba wax and candelilla wax is used in the cosmetic composition of the present invention. This allows the obtainment of the ideal and necessary density, texture and hardness for the end performance of the product. Furthermore, the combination of these waxes in the ideal concentration allows for a more intense shine and color without providing any opacification of the emulsion.

A preferred shine and performance improvement promoting complex comprises carnauba wax and candelilla wax in a quantity that varies from 8% to 15% of carnauba wax and a quantity that varies from 10% to 13% of candelilla wax by weight, based on the total weight of the composition.

The ideal quantity of each one of the waxes in the above combination should be the minimum effective in order not to promote the drying of the base or of the actual composition, to not form lumps and to provide volume.

The refining effects of the ideal drying time and the structure of the most resistant, homogenous and flexible film network obtained by the present invention are due to the presence of the two principal complexes:
a) a film forming complex; and
b) a shine and performance improvement promoting complex of the end product.

### - Carrier

A third essential component of the present invention is a cosmetically acceptable carrier. Water is the base of several possibilities of cosmetic compositions as the object of the present invention. The compositions of the present invention comprise, preferentially, demineralized or distilled water in a suitable percentage (q.s.p.) in order to achieve 100% of the formula, based on the total weight of the present composition.

### - Emulsifying system:

The present invention comprises an oil-in-water emulsion. This type of emulsion allows the product to be applied easily, with an ideal drying time and without the formation of lumps that normally occur in anhydrous (without water) formulas or in water-in-oil emulsions. The development of new and more effective film forming agents for the abovementioned water phase allow the emulsion of the present invention to achieve one of the most desired characteristics of the anhydrous formulas, which is the long duration and the capacity of not smudging throughout the day.

Furthermore, an oil-in-water emulsion is easily removed with soap and water or makeup remover without the need to rub the area excessively. This does not happen with water-in-oil emulsions or anhydrous formulations.

Preferentially, the emulsifying system of the cosmetic composition of the O/W emulsion type for the keratinic material, object of the present invention, is constituted by one or more amphiphilic emulsifying agents. Preferentially, the amphiphilic emulsifying agent is selected from a group that consists of: triethanolamine stearate, glyceryl stearate, PEG-40 stearate, lecithin and polysorbate 20.

In a most preferred embodiment, an emulsifying system is used that is constituted of triethanolamine stearate in a quantity that varies from 5% to 10% by weight, based on the total weight of the composition.

As well as the abovementioned components, the cosmetic composition of the present invention can be comprised of other ingredients in order to provide preferred and more suitable results to the desired practical use of the end product.

### - Thickening agent

The thickening agent employed in the cosmetic composition of the present invention can be selected from the group: hydroxyethyl cellulose, gum arabic, xanthan gum, gums recognized as thickeners, carboxymethyl cellulose, carbopol and silicates, and mixtures thereof, among others already recognized and used for this operation in similar cosmetic compositions, such as, for example, mascara for eyelashes.

Preferentially, the thickening agents used in the formulation of the cosmetic composition of the present invention are:
- Hydroxyethyl cellulose:
   Hydroxyethyl cellulose is a cellulose derivative capable of providing the formation of a network in the dispersion carrier when adequately hydrated. The principal impact of the network formed by this polymer is in the rheological profile of the composition, making the product more moldable. Furthermore, the hydroxyethyl cellulose is capable of providing the formation of a more flexible film and also supplies a larger deposit of end product on the keratinic fiber.
   Hydroxyethyl cellulose, when used together with the waxes and another thickener such as gum arabic, is responsible for the quantity of the product to be deposited and an imbalance of the concentrations of these ingredients can result in formation of the lumps by excessive deposit of the formulation or the obtainment of a brittle film that over time detaches from the hairs and appears as black spots. Controlling the quantities of these components combined, the deposit of the formula can also be controlled, allowing a greater perception of the volume and definition of the eyelashes.

The network formed by the hydroxyethyl cellulose together with another hydrosoluble or hydrodispersible thickening agent, for example, gum arabic, allows the adequate envelopment of each hair with the deposited film.

### - Gum arabic:

Gum arabic is a polysaccharide containing variable quantities of D-galactose, L-arabinose, L-ramnose and other derived acids, such as D-glucuronic acid and 4-O-methyl-D-glucuronic acid and multiple glycoproteins.

Gum arabic is a natural resin composed of polysaccharides and glycoproteins, which are extracted from two species of acacia from the sub-Sahara region, more specifically from the species *Acacia senegal* and *Acacia seyal.*

Gum arabic is a thickener of the water phase that acts by the formation of a three dimensional network, which is capable of enveloping each hair uniformly and cohesively, providing definition and volume. The gum assists in the formation of a more resistant film.

In the preferred embodiments of the present invention, a thickening agent and a rheological agent are used, preferentially chosen from hydroxyethyl cellulose, gum arabic, xanthan gum and carboxymethyl cellulose, more preferentially a combination of hydroxyethyl cellulose and xanthan gum in a quantity that varies from 0.2% to 3.5% by weight, based on the total weight of the cosmetic composition.

### Other optional components

Optionally, the cosmetic composition, object of the present invention, comprises cosmetic adjuvants selected from the group:
- Conditioning agents such as polyquartenium-10, panthenol;
- Pigments such as Black NF, among others;
- Preserving agents such as phenoxyethanol, among others;
- Antioxidizing agents such as tocopheryl acetate, PEG-8, ascorbyl palmitate, ascorbic acid and citric acid, among others;
- Emollient agents such as extract of *Lonicera caprifolium,* among others;
- Humectant agents such as propylene glycol, among others.

The cosmetic composition of the present invention is particularly useful for use as a mascara for eyelashes and advantageously presents suitable and safe physical and chemical characteristics to the user of the product, for example:
1. It is stable for a period of at least two years;
2. It presents a suitable texture during the application, homogenous, non-crumbling and without the formation of lumps;
3. It is easy to apply;
4. It does not present allergenicity:
5. Because it does not provoke irritation to the skin or the eyes, it is more comfortable and allows daily use or even more than once per day;
6. It presents suitable chemical stability;
7. It provides a long lasting duration;
8. It presents an ideal drying time and more resistant film;
9. It has an ideal texture that forms a homogenous and flexible film (it is homogenous and flexible), allowing the application of the end product on the eyelashes and other hairs in a cohesive and uniform manner;
10. It is non-crumbling and does not form lumps;
11. It can be used with different types of comb or brush applicators;
12. It can be used to lengthen the eyelashes or to give volume to them, depending on the eyelash comb used;
13. It can be applied after a primer or on its own;
14. It is easily applied and adheres to hairs as well as to the eyelashes;
15. It does not smudge during daily use;
16. It is easily removed;

A preferred embodiment of the cosmetic composition, object of the present invention, is a mascara presenting a pH range of (7-8), close to the pH of a tear, which produces less irritation.

Preferred embodiments of the present invention are: a mascara for the eyelashes in order to color, lengthen or give volume to the eyelashes and a mascara for hair in order to provide a temporary tint. For example, the mascara for the eyelash can be colorless or comprise coloring or pearlescent pigments.

In a preferred embodiment, depending on the type of comb chosen, the composition, object of the present invention, can be used to lengthen the eyelashes or to give volume to them.

The following presented illustrative examples serve to better describe the present invention. However, the data and illustrated procedures merely refer to some embodiments of the present invention and should not be considered as limiting factors in the scope of the invention.

### 1 - Example of the composition of mascara for eyelashes

The following table 1 presents the composition of mascara for eyelashes in accordance with the present invention.

**Table 1:**

| Raw materials | Function | Quantity (%m/m) |
|---|---|---|
| Glyceryl stearate | Emulsifier | 1 |
| Lecithin | Emulsifier | 0.3 |
| Glycol propylene | Humectant | 1.5 |
| Water | Carrier | QSP |
| PVP/eicosane copolymer | Film forming agent | 1.5 |
| Panthenol | Conditioning and nutritional agent | 0.5 |
| Hydroxyethyl cellulose | Rheological agent / thickening agent | 0.45 |
| Senegalese gum arabic (gum arabic) | Rheological agent / thickening agent | 2.5 |
| Tocopheryl acetate (vitamin E) | Antioxidant | 0.5 |
| Polyquartenium-10 | Conditioning agent | 0.1 |
| Polysorbate 20 | Co-emulsifier | 0.1 |
| Peg-8, tocopherol, ascorbyl palmitate, ascorbic acid, citric acid | Antioxidant | 0.1 |
| Peg-40 Stearate | Co-emulsifier | 0.3 |
| Cl 77499 | Pigment | 7 |
| Phenoxyethanol | Preservative | 0.8 |
| Triethanolamine | pH adjuster and base for the saponification reaction | 5.5 |
| Stearic acid | Emulsifier | |
| Candelilla wax | Shine promoter agent | 4 |
| Carnauba vegetable wax | Shine promoter agent | 4 |
| Copolymer acrylate | Film forming agent | 10 |
| *Lonicera caprifolium* extract | Emollient (preservative) | 0.3 |

### Manufacturing process of the composition of example 1

1) Water, propylene glycol and triethanolamine are added to a main reactor,;
2) The mixture obtained above is homogenized until the liquids are completely mixed;
3) The polyquartenium-10 and the hydroxyethyl cellulose are added to the reactor;
4) Homogenize until the total dispersion of the polymers without any lumps;
5) Then gum arabic is added and the mixture is once again fully homogenized ;
6) After the formation of the gel without lumps, the pigment is added. The dispersion should be performed carefully in order not to have lumps of pigment in the end product. The use of a rotor-stator homogenizer is recommended;
7) After the dispersion is complete, this phase is heated up to 85 to 90°C;
8) In parallel, all the components of the oil phase (PVP/eicosane copolymer, glyceryl stearate, PEG-40 stearate, polysorbate 20, vitamin E, stearic acid, waxes, lecithin and the antioxidant are added to an auxiliary reactor. This phase is also heated up to 85 to 90°C;
9) When both phases have been heated the oil phase is transferred to the main reactor;
10) Homogenize for 20 minutes;
11) Acrylate copolymer is added at a temperature below 60°C;
12) Fully Homogenize the mixture thus obtained;
13) At a temperature below 40°C, the panthenol is added and soon followed by the addition of the phenoxyethanol and the *Lonicera caprifolium* extract;
14) The resultant mixture is homogenized once again until a brilliant and homogenous emulsion is produced.

### 2 - Test examples

### a) Rheological Analysis

Figure 1 shows the results of rheological analysis of a sample of mascara composition of Example 1 performed on the equipment Brookfield R / S Plus Rheometer. For this analysis a cone C50-1 was used..

Initially a low shear rate (two minutes) was applied to the sample and then it was significantly increased for thirty seconds in order to challenge a structure of the sample. The lowest shear rate was applied again and it was observed that the product returned to its original physical characteristics.

Therefore, through this analysis it is possible to make an analogy with possible kinds of shear that the product may suffer, such as when submitted to the process of filling the recipient and to the brush applicator. This comparison shows that even after undergoing shear by the brush friction or the container filling process, the product returns to its original structure and therefore maintains its performance.

### b) Acceptability test of mascara composition of Example 1 with ophthalmic accompaniment

A study of 35 volunteers was performed. They were female (in accordance with the type of product), of different races, with ages from 21 to 56 years old and users of cosmetic products, who were approved in accordance with the exclusion and inclusion criteria of the test. The exclusion criteria comprised, for example, localized dermatological diseases, including the periocular region, or generalized, and pregnancy or lactation.

During the study, in real conditions of use, following the frequency and method of application, no volunteer presented any type of adverse ocular or cutaneous reaction in relation to the product.

### b) Mono-blind clinical study of the cutaneous and ocular region tolerance, in real conditions of use of the product:

- Objective of the test: To determine the prevalence of adverse reactions in real conditions of use of the product
- Methodology and study group:
- Population and sample / volunteer selection: Female volunteers were selected, of all races and aged between 18 to 60 years old.

The exclusion criteria comprised localized dermatological diseases, including in the periocular region, or generalized, and pregnancy or lactation.

The volunteers were submitted to an interview that included a consent form to be signed by the volunteer that wished to participate in the study, information about product use, clinical testing and dermatological examination.

The sampling included 35 volunteers between the ages of 21 to 56 years old (average age of 37 years old).

### - Study period:

The research was conducted over a period of 21 days.

### - Methodology:

After the selection, the volunteers were instructed to use the product for 3 weeks, following the instructions supplied by the company, as follows:
- Keep the product well closed;
- Remove any excess of the product from the brush;
- Apply the product on the upper eyelashes with gentle movements without excessively pressing on the application area;
- Remove the product using cotton moistened in a standard makeup remover;
- Shake before using and then rinse with water;
- Use daily;
- Keep out of reach of children;
- Do not use other cosmetic products in the regions of the application of the product during the research;
- Communicate immediately any reactions or symptoms;
- In the case of cutaneous reactions, the appearance of the patient was requested in order to perform a dermatological examination and to take photographs;
- The patients were instructed with respect to the completion of a mini-diary (day and time of the product application) and to use the product daily;
- During this period, the volunteers were supervised by a dermatologist and an ophthalmologist. They were guided to seek out immediately the coordinator of the research in the case of the appearance of any adverse sign and/or symptom of use.

### - Evaluations:

Dermatological clinical evaluation: The volunteers were supervised by a dermatologist during the entire research and evaluated in the case of the appearance of any symptom or sign, for confirmation of the actual and correct use of the product and detection of possible adverse reactions.

The volunteers were instructed to seek out the coordinator of the research at any time, in the case of the appearance of any adverse reactions. In these cases, they were sent for evaluation and guidance to the responsible dermatologist, who performed the examination, classification of the reaction and the realization of the appropriate solution (guidance and/or medication and photographic documentation, when necessary).

### - Adverse cutaneous reactions:

The adverse cutaneous reactions were classified in the following manner (SOTER & FITZPATRICK, 1979; BAER & GIGLI, 1979):
Contact dermatitis: Characterized by the presence of one or more of the following symptoms or signs: intense itching, erythema, edema, scaling, papules or vesicles and divided into two categories: primary irritation and cutaneous sensitivity.
Acneiform reaction: The appearance of any one of the clinical lesions of acne: comedones, papules, pustules, nodules or cysts during the use of the product, which disappear after the suspension of use and without any other possible related cause (hormonal alteration and use of medication, among others).
Urticarial reaction: The appearance of ephemeral lasting erythematous-edematous plaque in the areas of the product application.
Pigmentation disturbances: Hypochromic or hyperchromic stains that appear after the start of the use of the product, without any other defined cause.
Unsuitable use of the product / insufficient information: Any reaction arising from the different use from that instructed and that disappears when the individual begins to follow the correct use.

All the reactions were classified according to their intensity as light, moderate or intense. When necessary, the use of the product was interrupted.
- Patch test: The volunteers that presented adverse reaction of the contact dermatitis type were submitted to epicutaneous tests. The evaluation scale used was that recommended by the International Research Contact Dermatitis Group (IRCDG) (FISHER, 1995), as follows:
- Reaction X Result:
   0- absent negative (-)
   1- light doubtful erythema (?)
   2- clear positive erythema (+)
   3- erythema + edema + positive papules (++)
   4- erythema + edema + papules + positive vesicles (+++)

### Ophthalmological Clinical Evaluation

The volunteers were supervised by an ophthalmologist during the entire research and evaluated in the case of the appearance of any symptom or sign, for confirmation of the actual and correct use of the product and detection of possible adverse reactions.

The volunteers were instructed to seek out the coordinator of the research, at any time, in the case of the appearance of any adverse reactions. In these cases, they were sent for evaluation and guidance to the responsible ophthalmologist, who performed the examination, classification of the reaction and the realization of the appropriate solution (guidance and/or medication and photographic documentation, when necessary).

### - Adverse Ocular Reactions:

The adverse ocular reactions were evaluated in accordance with the following specifications:
a) Palpebral edema: 0 - absent; 1 - light; 2 - moderate; and 3 - severe.
b) Orbicular secretion: 0 - normal; 1 - excessive humidity; 2 - isolated secretion; 3 - thick secretion.
c) Bulbar conjunctiva: 0 - normal; 1 - light hyperemia; 2 - moderate hyperemia; 3 - intense hyperemia.
d) Palpebral conjunctiva: 0 - normal; 1 - light hyperemia; 2 - moderate hyperemia; 3 - intense hyperemia.
e) Cornea: 0 - normal; 1 - light keratitis; 2 - moderate keratitis; 3 - intense keratitis.
f) Burning sensation: 0 - absent; 1 - light; 2 - moderate; e 3 - intense.

Ocular irritability: 0,0 - absent; 0,0 < 10 = 1,0; 1,0 < IO = 2,5; 2,5 < IO = 3,5; e IO > 3,5.

The ocular irritability indices were calculated by adding up the values found in each one of the items (a) - (f), and dividing by the number of volunteers.

### - Design of the study:

The study was a mono-blind clinical trial.

### - Results:

- Adherence to the study: The 35 volunteers completed the study.
- Dermatological clinical evaluation: During the study, no volunteer presented any type of adverse cutaneous reaction related to the use of the product.
- Ophthalmological clinical evaluation: During the study, no volunteer presented any type of adverse ocular reaction related to the use of the product.

The results are displayed below, in the format of table 2 (dermatological clinical evaluation) and table 3 (ophthalmological clinical evaluation) of the specifications of the present invention:

**Table 2:**

| Volunteer | T0 | T7 | T14 | T21 |
|---|---|---|---|---|
| 1 | 0 | 0 | 0 | 0 |
| 2 | 0 | 0 | 0 | 0 |
| 3 | 0 | 0 | 0 | 0 |
| 4 | 0 | 0 | 0 | 0 |
| 5 | 0 | 0 | 0 | 0 |
| 6 | 0 | 0 | 0 | 0 |
| 7 | 0 | 0 | 0 | 0 |
| 8 | 0 | 0 | 0 | 0 |
| 9 | 0 | 0 | 0 | 0 |
| 10 | 0 | 0 | 0 | 0 |
| 11 | 0 | 0 | 0 | 0 |
| 12 | 0 | 0 | 0 | 0 |
| 13 | 0 | 0 | 0 | 0 |
| 14 | 0 | 0 | 0 | 0 |
| 15 | 0 | 0 | 0 | 0 |
| 16 | 0 | 0 | 0 | 0 |
| 17 | 0 | 0 | 0 | 0 |
| 18 | 0 | 0 | 0 | 0 |
| 19 | 0 | 0 | 0 | 0 |
| 20 | 0 | 0 | 0 | 0 |
| 21 | 0 | 0 | 0 | 0 |
| 22 | 0 | 0 | 0 | 0 |
| 23 | 0 | 0 | 0 | 0 |
| 24 | 0 | 0 | 0 | 0 |
| 25 | 0 | 0 | 0 | 0 |
| 26 | 0 | 0 | 0 | 0 |
| 27 | 0 | 0 | 0 | 0 |
| 28 | 0 | 0 | 0 | 0 |
| 29 | 0 | 0 | 0 | 0 |
| 30 | 0 | 0 | 0 | 0 |
| 31 | 0 | 0 | 0 | 0 |
| 32 | 0 | 0 | 0 | 0 |
| 33 | 0 | 0 | 0 | 0 |
| 34 | 0 | 0 | 0 | 0 |
| 35 | 0 | 0 | 0 | 0 |

**Table 3:**

| Volunteer | T0 | T7 | T14 | T21 |
|---|---|---|---|---|
| 1 | 0 | 0 | 0 | 0 |
| 2 | 0 | 0 | 0 | 0 |
| 3 | 0 | 0 | 0 | 0 |
| 4 | 0 | 0 | 0 | 0 |
| 5 | 0 | 0 | 0 | 0 |
| 6 | 0 | 0 | 0 | 0 |
| 7 | 0 | 0 | 0 | 0 |
| 8 | 0 | 0 | 0 | 0 |
| 9 | 0 | 0 | 0 | 0 |
| 10 | 0 | 0 | 0 | 0 |
| 11 | 0 | 0 | 0 | 0 |
| 12 | 0 | 0 | 0 | 0 |
| 13 | 0 | 0 | 0 | 0 |
| 14 | 0 | 0 | 0 | 0 |
| 15 | 0 | 0 | 0 | 0 |
| 16 | 0 | 0 | 0 | 0 |
| 17 | 0 | 0 | 0 | 0 |
| 18 | 0 | 0 | 0 | 0 |
| 19 | 0 | 0 | 0 | 0 |
| 20 | 0 | 0 | 0 | 0 |
| 21 | 0 | 0 | 0 | 0 |
| 22 | 0 | 0 | 0 | 0 |
| 23 | 0 | 0 | 0 | 0 |
| 24 | 0 | 0 | 0 | 0 |
| 25 | 0 | 0 | 0 | 0 |
| 26 | 0 | 0 | 0 | 0 |
| 27 | 0 | 0 | 0 | 0 |
| 28 | 0 | 0 | 0 | 0 |
| 29 | 0 | 0 | 0 | 0 |
| 30 | 0 | 0 | 0 | 0 |
| 31 | 0 | 0 | 0 | 0 |
| 32 | 0 | 0 | 0 | 0 |
| 33 | 0 | 0 | 0 | 0 |
| 34 | 0 | 0 | 0 | 0 |
| 35 | 0 | 0 | 0 | 0 |

The times (T) were measured in days. T0, T7, T14 and T21 refer to the evaluations performed on the first day and later on the seventh, fourteenth and twenty-first days, respectively.

Conclusion: In accordance with the methodology used to evaluate the prevalence of adverse reactions in real conditions of use of the product, it can be concluded that in such real conditions of use, following the already mentioned pre-determined frequency and mode of application, no volunteer presented cutaneous or ocular lesions related to the use of the product.

## Claims

1. A cosmetic composition containing an oil-in-water emulsion, **characterized by** comprising:
a) from 1% to 25 % by weight, based on the total weight of the composition, of a film forming complex comprising of at least one hydrosoluble or hydrodispersible film forming agent and at least one liposoluble or lipodispersible film forming agent;
b) from 8% to 15% in weight, based on the total weight of the composition, of a shine and performance improvement promoting complex; and
c) a cosmetically acceptable carrier.

2. A cosmetic composition according to claim 1, **characterized in that** the concentration of the film forming complex is from 15 to 20% in weight, based on the total weight of the composition.

3. A cosmetic composition according to claim 1 or 2, **characterized in that** the hydrosoluble or hydrodispersible film forming agent is selected from the group comprising acrylic/acrylate copolymer, methacrylate copolymer, PVP/eicosane copolymer, sodium polyacrylate, ammonium polyacrylate, or a mixture of these.

4. A cosmetic composition according to claim 3, **characterized in that** the hydrosoluble or hydrodispersible film forming agent is selected from acrylate copolymer, PVP/eicosane copolymer, or a mixture of these.

5. A cosmetic composition according to claim 4, **characterized in that** the hydrosoluble or hydrodispersible film forming agent is the acrylate copolymer.

6. A cosmetic composition according to any one of claims 1 to 5, **characterized in that** the liposoluble or lipodispersible film forming agent is selected from the group comprising PVP/eicosane copolymer, trimethylsiloxysilicate, polyurethanes, or a mixture of these.

7. A cosmetic composition according to claim 6, **characterized in that** the liposoluble or lipodispersible film forming agent is the PVP/eicosane copolymer.

8. A cosmetic composition according to any one of claims 1 to 7, **characterized in that** the shine and performance improvement promoting complex comprises a mixture of solid lipid components selected from vegetable, animal or synthetic waxes.

9. A cosmetic composition according to claim 7, **characterized by** comprising a combination of solid lipid components selected from beeswax, ozokerite wax, sugar cane wax, ceresine wax, microcrystalline wax, carnauba wax, candelilla wax, or a mixture of these, and a glycolic material, propylene glycol and polyquartenium, or a mixture of these.

10. A cosmetic composition according to any one of claims 1 to 9, **characterized by** comprising from 0.2% to 3.5% by weight of a thickening agent.

11. A cosmetic composition according to claim 7, **characterized in that** the thickening agent is selected from the group that consists of hydroxyethyl cellulose, gum arabic, xanthan gum, carboxymethyl cellulose, carbopol, silicates, or their mixtures.

12. A cosmetic composition according to any one of the claims 1 to 11, **characterized in that** it comprises from 5% to 10% by weight of an emulsifying system.

13. A cosmetic composition according to claim 12, **characterized in that** the emulsifying system comprises at least one amphiphilic emulsifying agent selected from the group that consists of triethanolamine stearate, glyceryl stearate, PEG-40 stearate, lecithin and polysorbate 20.

14. A cosmetic composition according to any one of the claims 1 to 13, **characterized in that** the cosmetically acceptable carrier is water.

15. A cosmetic composition according to any one of the claims 1 to 14, **characterized by** further comprising a cosmetic adjuvant selected from the group that consists of conditioning agents, pigments, preserving agents, antioxidant agents, emollient agents and humectant agents.

16. A mascara for eyelashes, **characterized by** comprising a composition as defined in any one of claims 1 to 15.

17. A mascara for eyelashes according to claim 16, **characterized by** being colorless.

18. A mascara for eyelashes according to claim 16, **characterized by** comprising a coloring or pearlescent pigment.
